# EUROPEAN PATENT SPECIFICATION

(11) **EP 0 643 979 B1**
(45) Date of publication and mention of the grant of the patent: **25.10.2000**
(21) Application number: 94202390.4
(22) Date of filing: 22.08.1994
(51) Int. Cl.: A61M 25/00

(54) **Microcatheter**
Mikrokatheter
Microcatheter

(30) Priority: 22.09.1993 NL 9301642
(43) Date of publication of application: 22.03.1995
(73) Proprietor: CORDIS EUROPA N.V., NL-9301 LJ Roden (NL)
(72) Inventor: Van Muiden, Johannes Gerardus Maria, 9321 AZ Peize (NL)
(74) Representative: 't Jong, Bastiaan Jacobus

(56) References cited:
- EP-A- 0 063 859
- WO-A-91/17782
- WO-A-94/07549
- US-A- 4 563 180
- US-A- 4 983 169
- US-A- 5 061 257
- US-A- 5 085 649

## Description

The invention relates to a catheter as defined in the introductory part of claim 1. Such a catheter is known from US-A-5 061 257.

In this known catheter the portion at the distal end of the basic body, also called the tip portion, has a smaller diameter than the basic body and an increased flexibility. This is achieved in that only the flexible layer of material tends into the tip portion. This known catheter specifically is an angiographic catheter. The relatively flexible tip portion is favourable for preventing trauma to the patient.

The invention has for its object to provide a catheter having the features of the introductory portion of claim 1, also for preventing trauma to the patient, but being specifically intended for use in the tortuous, small blood vessels in the human brain. At the distal end such a catheter should be pliable to such an extent that it can pass through very small bends, smaller than 5 mm, in the vascular system of the brain. At the same time the catheter should be sufficiently stiff towards the proximal end to transmit the longitudinal forces when introducing and manipulating the catheter. Furthermore, these qualities must be combined in a catheter of which the distal end must be capable of penetrating into very narrow blood vessels with a diameter considerably smaller than 1 mm.

The microcatheter according to the invention as characterised in claim 1 has the properties with which these aims can be achieved.

During catheterization the catheter is introduced into a relatively wide blood vessel and advanced from there to ever narrower vessels. Towards the proximal end the thickness of the catheter may therefore be greater, without limiting the accessibility of the very small blood vessels. Because of the somewhat greater thickness close to the proximal end, the microcatheter can be manipulated properly.

A desired increase in the pliability towards the distal end is already achieved due to a decrease in the diameter but is additionally enhanced, by using relatively stiff material close to the proximal end and increasingly, rather more pliable material towards the distal end. In that way a desired, great difference in flexibility of the distal end and the proximal end can be achieved.

In order to be able to use the catheter according to the invention with a metal guide wire or one coated with polytetrafluorethylene, which obviously will have a very small diameter, the measure as characterised in claim 3 is preferably employed. The friction between the guide wire and the catheter will consequently be so little that, even in case of a very tortuous passage of the catheter in the vascular system in the brain, the guide wire will not get stuck inside the catheter. The layer of material characterised by low friction can for instance be made of polytetrafluorethylene or polyethylene, which at the same time can withstand aggressive chemotherapeutics or wear from the guide wire.

In order to achieve a good pressure resistance and a good torsional resistance at least close to the proximal end, the measure as characterised in claim 3 is preferably employed.

To reduce axial flexibility at the distal end, claim 4 is employed in an advantageous manner.

The microcatheter according to the invention will have for the intended application preferably the dimensions as characterised in claim 5.

The invention will be explained in greater detail with reference to four examples of embodiments shown schematically in the attached drawings.

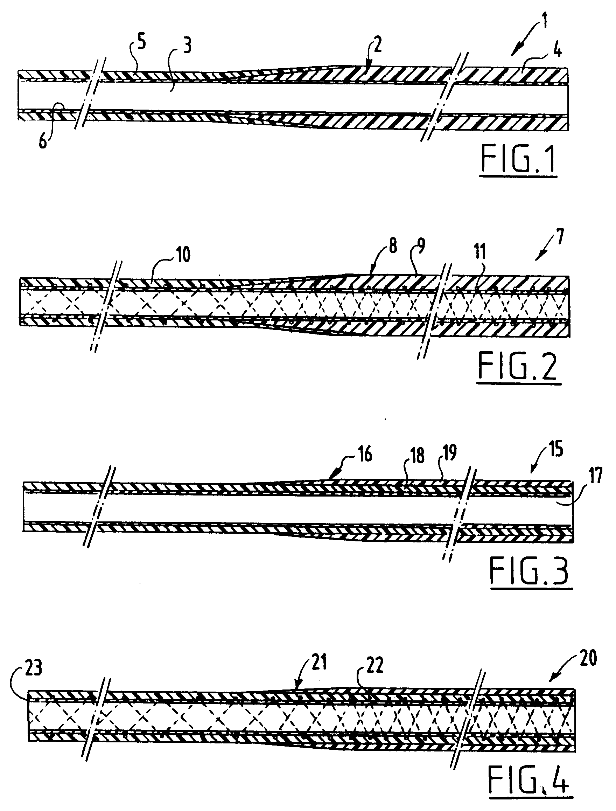

Each of the figures 1 up to and including 4 illustrates schematically a basic body of a catheter according to the invention, with the proximal end on the right hand side and the distal end on the left hand side.

The basic body 2 of the catheter 1 shown in fig. 1 comprises one lumen 3 which runs from the proximal end, to which a connecting member, not shown here, has been attached, to the distal end. As the figure illustrates, the diameter of the basic body 2 decreases from the proximal end towards the distal end.

The basic body 2 is made up of a relatively stiff layer of material 4 and a relatively flexible layer of material 5. The cross-section of the stiff layer of material 4 decreases in relation to the flexible layer of material 5 in a transition zone. This transition zone can have a length of several tens of centimetres, for instance something like 50 cm. Close to the proximal end the stiff layer of material 4 has a constant cross-section and close to the distal end the flexible layer of material 5 has a constant cross-section.

The gradual transition from the one layer of material 4 to the other 5, can be achieved by manufacturing the basic body 2 by intermittent extrusion. The supply of the stiff material 4 is gradually reduced, while the supply of the flexible material 5 is gradually increased. In order to achieve the reduction in diameter, the reduction in the supply of the stiff material 4 will however be set at less than the increase in the supply of the flexible material 5.

The reduction in outside diameter from the proximal towards the distal end can also be achieved by increasing the carrying velocity of the extrusion product during the transition from the stiff to the flexible material.

The lumen 3 is bounded by a layer 6 made of a low friction material, such as polytetrafluorethylene or polyethylene, in order to achieve minimal friction as regards a guide wire to be advanced through the lumen 3.

The catheter 7 as shown in fig. 2 is basically similar to the catheter 1 as shown in fig. 1. Also in this case the basic body 8 comprises one lumen, made of a stiff layer of material 9 and a flexible layer of material 10, wherein towards the distal end the diameter of the basic body 8 decreases.

The catheter 7 comprises furthermore a braided or wound reinforcing layer 11 of metal or plastic threads or filaments. As the figure illustrates, the pitch of the braided or wound reinforcing layer is smaller at the proximal end than at the distal end, but, with another embodiment, one can also opt for the pitch to be constant from the proximal to the distal end of the catheter. In this case the word pitch is understood to mean the distance between the threads or filaments. By using a smaller pitch close to the proximal end, the number of threads per unit length is greater, so that the reinforcing layer contributes more to the torsional resistance of the catheter than at the distal end. At the distal end the reinforcing layer contributes more to the reduction in flexibility due to the larger pitch.

The catheter 15 illustrated in fig. 3 comprises a basic body 16 which has been made slightly different from the catheter 1 in fig. 1. The catheter 15 also comprises one lumen 17, a flexible layer of material 18 and a stiff layer of material 19. However, the thickness of the flexible layer of material 18 is constant over the entire length of the basic body 16 whereas the thickness of the stiff layer of material 19 reduces to zero towards the distal end. Consequently a change in the flexibility and the thickness of the catheter is achieved. The lumen is bounded by a layer with good frictional properties like for instance polytetrafluorethylene or polyethylene.

The catheter 20 of fig. 4 has basically the same construction again as the one in fig. 3, wherein however once again a reinforcing layer 22 has been incorporated in the basic body 21 of which the threads can have an increasing pitch towards the distal end.

In the catheter 20 a layer 23 with good frictional properties has been applied. As has been mentioned before, this layer is preferably a polytetrafluorethylene or polyethylene layer.

For the stiff layer of material a suitable polyurethane or a low pressure polyethylene can be chosen, while for the flexible layer also a polyurethane, but in that case of a composition which displays little stiffness, or a polyethylene with a vinyl acetate can be chosen.

With the invention a microcatheter, suitable for the application as described, of considerable length and a very small diameter of less than 1 mm, can be manufactured. From the proximal end to the distal end the diameter can decrease from substantially 1 mm to substantially 0,7 mm. The wall thickness of the catheter can in that case be in the order of 0,1 mm, so that the inside diameter of the lumen can be something like 0,5 mm.

## Claims

1. Catheter (1) comprising a tube-like basic body (2) with a proximal end and a distal end, at least one lumen (3) extending from the proximal to the distal end, for which a connecting member has been applied, a portion at the distal end having a smaller diameter than the basic body, said basic body comprising a stiff (4) and a flexible (5) layer of material over at least part of its length, **characterized in that** the flexible layer of material has a constant or increasing thickness and the stiff layer of material has a decreasing thickness in the direction of the proximal to the distal end, such that the diameter of the basic body gradually decreases and the flexibility thereof gradually increases in this direction over a length of several tens of centimeters and in that the catheter (1) is a micro catheter, said part having a diameter of substantially 1 mm or less.

2. Microcatheter as claimed in claim 1, wherein the lumen (3) is bounded by a layer (6) of low friction material.

3. Microcatheter as claimed in one of the previous claims, comprising a reinforcing layer (11; 22) braided or wound of threads or filaments.

4. Microcatheter as claimed in claim 3, wherein the pitch of the braided or wound threads or filaments increases towards the distal end.

5. Microcatheter as claimed in one of the previous claims, wherein the diameter decreases from 1,0 or 0,8 mm to 0,75 or 0,65 mm.

## Patentansprüche

1. Katheter (1) mit einem rohrartigen Grundkörper (2) mit einem proximalen Ende und einem distalen Ende, wenigstens einem Lumen (3), das sich von dem proximalen zu dem distalen Ende, an dem ein Verbindungselement verwendet wird, erstreckt, wobei ein Abschnitt am distalen Ende einen kleineren Durchmesser als der Grundkörper hat und der Grundkörper über wenigstens einen Teil seiner Länge eine steife (4) und eine flexible (5) Materialschicht umfaßt, dadurch gekennzeichnet, daß die flexible Materialschicht eine konstante oder zunehmende Dicke und die steife Materialschicht eine abnehmende Dicke in Richtung des proximalen Endes zu dem distalen Ende hat, derart, daß der Durchmesser des Grundkörpers allmählich abnimmt und dessen Flexibilität allmählich über eine Länge von mehreren 10 cm in dieser Richtung zunimmt und daß der Katheter (1) ein Mikrokatheter ist, wobei dieser Teil einen Durchmesser von im wesentlichen 1 mm oder geringer hat.

2. Mikrokatheter nach Anspruch 1, wobei das Lumen (3) begrenzt ist durch eine Schicht (6) aus einem friktionsarmen Material bzw. Material geringer Reibung.

3. Mikrokatheter nach einem der vorstehenden Ansprüche, mit einer Verstärkungsschicht (11; 22) die geflochten oder gewunden ist aus Verästelungen oder Fasern bzw. Drähten.

4. Mikrokatheter nach Anspruch 3, wobei der Gang der geflochtenen oder gewundenen Verästelungen oder Fasern bzw. Drähte in Richtung des distalen Endes zunimmt.

5. Mikrokatheter nach einem der vorstehenden Ansprüche, wobei der Durchmesser von 1,0 oder 0,8 mm auf 0,75 oder 0,65 mm abnimmt.

## Revendications

1. Cathéter (1) comprenant un corps de base (2) de forme tubulaire ayant une extrémité proximale et une extrémité distale, au moins une lumière (3) s'étendant de l'extrémité proximale à l'extrémité distale, à laquelle a été appliqué un élément de connexion, une portion à l'extrémité distale ayant un diamètre inférieur à celui du corps de base, ledit corps de base comprenant une couche de matière rigide (4) et une couche de matière souple (5) sur au moins une partie de sa longueur, caractérisé en ce que la couche de matière souple a une épaisseur constante ou croissante et la couche de matière rigide a une épaisseur décroissante de l'extrémité proximale à l'extrémité distale, de sorte que le diamètre du corps de base diminue progressivement et la souplesse de celui-ci augmente progressivement dans ce sens sur une longueur de quelques dixièmes de centimètre et en ce que le cathéter (1) est un microcathéter, ladite partie ayant un diamètre de sensiblement 1 mm ou moins.

2. Microcathéter selon la revendication 1, dans lequel la lumière (3) est délimitée par une couche (6) de matière de faible frottement.

3. Microcathéter selon l'une des revendications précédentes, comprenant une couche de renforcement (11; 22), tressée ou enroulée, de fils ou de filaments.

4. Microcathéter selon la revendication 3, dans lequel le pas des fils ou des filaments tressés ou enroulés augmente en direction de l'extrémité distale.

5. Microcathéter selon l'une des revendications précédentes, dans lequel le diamètre diminue d'une valeur égale à 1,0 mm ou à 0,8 mm à une valeur égale à 0,75 ou 0,65 mm.
